(19) Europäisches Patentamt
European Patent Office
Office européen des brevets

(11) Veröffentlichungsnummer: **0 045 450**
**B1**

(12) # EUROPÄISCHE PATENTSCHRIFT

(45) Veröffentlichungstag der Patentschrift: **15.02.84**

(21) Anmeldenummer: **81105833.8**

(22) Anmeldetag: **23.07.81**

(51) Int. Cl.³: **C 07 C 131/00,** C 07 C 103/50, A 61 K 31/15, A 61 K 31/195 // C07D243/24

(54) **Benzophenon-Derivate, Verfahren zu ihrer Herstellung, sowie diese enthaltende Arzneimittel.**

(30) Priorität: **31.07.80 CH 5840/80**

(43) Veröffentlichungstag der Anmeldung: **10.02.82 Patentblatt 82/6**

(45) Bekanntmachung des Hinweises auf die Patenterteilung: **15.02.84 Patentblatt 84/7**

(84) Benannte Vertragsstaaten: **AT BE CH DE FR GB IT LI LU NL SE**

(56) Entgegenhaltungen:
**EP - A - 0 000 332**
**DE - A - 1 618 518**
**DE - A - 2 327 715**
**GB - A - 1 144 516**

(73) Patentinhaber: **F. HOFFMANN-LA ROCHE & CO. Aktiengesellschaft, CH-4002 Basel (CH)**

(72) Erfinder: **Branca, Quirico, Dr., Lenzgasse 2, CH-4056 Basel (CH)**
Erfinder: **Fischli, Albert Eduard, Prof.,Dr., Am Ausserberg 20, CH-4125 Riehen (CH)**
Erfinder: **Szente, André, Dr., Baselstrasse 70, CH-4125 Riehen (CH)**

(74) Vertreter: **Lederer, Franz, Dr. et al, Patentanwälte Dr. Franz Lederer Dipl.-Ing. Reiner F. Meyer-Roxlau Lucile-Grahn-Strasse 22, D-8000 München 80 (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Benzophenon-Derivate, Verfahren zu ihrer Herstellung, sowie diese enthaltende Arzneimittel

Die vorliegende Erfindung betrifft neue Benzophenonoxim-Derivate der allgemeinen Formel

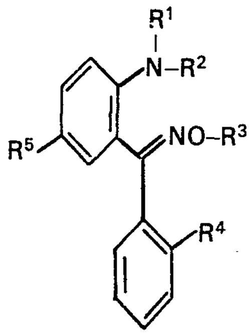

(I)

worin $R^1$ Wasserstoff oder niederes Alkyl, $R^2$ Wasserstoff oder Aminoacetyl, $R^3$ Wasserstoff oder niederes Alkyl, $R^4$ Wasserstoff oder Halogen, $R^5$ Wasserstoff, Amino, Nitro oder einen Rest der Formel $R^3$–ON=C($R^6$)– und $R^6$ niederes Alkyl bedeuten, mit der Massgabe, dass $R^5$ einen Rest der Formel H–ON=C($R^6$)– bedeutet, wenn $R^2$ und $R^3$ beide Wasserstoff sind, und pharmazeutisch annehmbare Säureadditionssalze von Verbindungen der allgemeinen Formel I.

Diese Verbindungen sind neu und zeichnen sich durch unerwartete wertvolle pharmakodynamische Eigenschaften aus.

Gegenstand der vorliegenden Erfindung sind Verbindungen der allgemeinen Formel I und ihre pharmazeutisch annehmbaren Säureadditionssalze als solche und als pharmazeutische Wirkstoffe, die Herstellung solcher Verbindungen und Arzneimittel, enthaltend eine Verbindung der allgemeinen Formel I oder ein pharmazeutisch annehmbares Säureadditionssalz davon, die Herstellung solcher Arzneimittel sowie die Verwendung von Verbindungen der allgemeinen Formel I und von pharmazeutisch annehmbaren Säureadditionssalzen davon bei der Bekämpfung bzw. Verhütung von Krankheiten.

Der Ausdruck «niederes Alkyl» bezeichnet geradkettige oder verzweigte gesättigte Kohlenwasserstoffreste mit höchstens 7, vorzugsweise höchstens 4 Kohlenstoffatomen, wie Methyl, Äthyl, n-Propyl, Isopropyl, n-Butyl, t-Butyl, usw. Der Ausdruck «Halogen» bedeutet Fluor, Chlor, Brom oder Jod.

Unter den Verbindungen der Formel I sind diejenigen bevorzugt, worin $R^1$ Wasserstoff oder Methyl bedeutet. Die bevorzugte Bedeutungsmöglichkeit von $R^2$ ist Aminoacetyl. $R^3$ bedeutet vorzugsweise Wasserstoff oder Methyl. Als Bedeutungsmöglichkeit von $R^4$ ist Wasserstoff, Fluor oder Chlor bevorzugt.

Eine ganz besonders bevorzugte Verbindung im Rahmen der vorliegenden Erfindung ist 2-Amino-2'-[o-fluor-α-(hydroxyimino)benzyl]-4'-[1-(hydroxyimino)äthyl]acetanilid.

Weitere im Rahmen der vorliegenden Erfindung bevorzugte, von der allgemeinen Formel I umfasste Verbindungen sind:

2-Amino-2'-[o-chlor-α-(hydroxyimino)benzyl]-4'-nitroacetanilid;

2-Amino-2'-[α-(hydroxyimino)benzyl]-4'-nitro-acetanilid;

2,4'-Diamino-2'-[o-chlor-α-(hydroxyimino) benzyl]-N-methyl-acetanilid;

2-Amino-2'-[o-fluor-α-(hydroxyimino)benzyl]-4'-nitroacetanilid;

2-Amino-2'-fluor-5-[1-(hydroxyimino)äthyl] benzophenon-oxim und

2-Amino-benzophenon-O-methyl-oxim.

Die neuen Benzophenonoxim-Derivate der allgemeinen Formel I und ihre pharmazeutisch annehmbaren Säureadditionssalze können erfindungsgemäss hergestellt werden, indem man

a) ein Benzodiazepin-Derivat der allgemeinen Formel

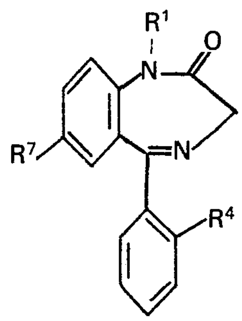

(II)

worin $R^7$ Wasserstoff, Amino, Nitro oder einen Rest der Formel $R^3$–ON=C($R^6$)– oder O=C($R^6$)– bedeutet, und $R^1$, $R^3$, $R^4$ und $R^6$ obige Bedeutung besitzen, mit einer Verbindung der allgemeinen Formel

$R^3$–$ONH_2$ III

worin $R^3$ obige Bedeutung besitzt, umsetzt, oder

b) eine Verbindung der allgemeinen Formel

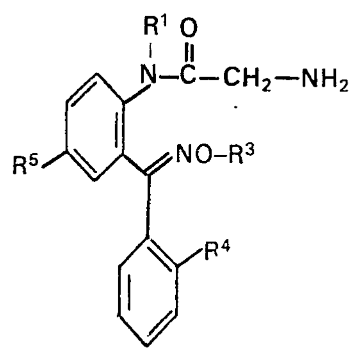

Ia

worin $R^1$, $R^3$, $R^4$ und $R^5$ obige Bedeutung besitzen, mit der Massgabe, dass $R^5$ einen Rest der Formel H–ON=C($R^6$)– bedeutet, wobei $R^6$ obige Bedeutung besitzt, wenn $R^3$ Wasserstoff ist, hydrolysiert, oder

c) eine Verbindung der allgemeinen Formel

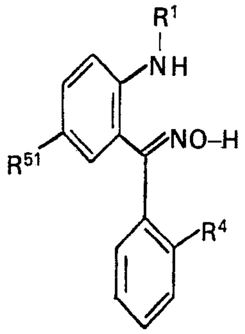

(Ib)

worin $R^{51}$ Wasserstoff, Amino, Nitro oder einen Rest der Formel $H–ON=C(R^6)–$ bedeutet, und $R^1$, $R^4$ und $R^6$ obige Bedeutung besitzen, am (an den) Oxim-Sauerstoffatom(en) alkyliert, oder

d) ein Benzophenon-Derivat der allgemeinen Formel

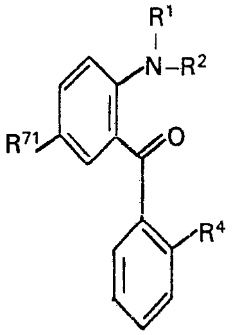

IV

worin $R^{71}$ Wasserstoff, Amino, Nitro oder einen Rest der Formel $O=C(R^6)–$ bedeutet, und $R^1$, $R^2$, $R^4$ und $R^6$ obige Bedeutung besitzen, mit einer Verbindung der allgemeinen Formel

$$R^3–ONH_2 \qquad III$$

worin $R^3$ obige Bedeutung besitzt, umsetzt, oder

e) ein Benzophenonoxim-Derivat der allgemeinen Formel I in ein pharmazeutisch annehmbares Säureadditionssalz überführt.

Gemäss Verfahrensvariante a) können die neuen Benzophenonoxim-Derivate der allgemeinen Formel I hergestellt werden, indem man Benzodiazepin-Derivate der allgemeinen Formel II mit Hydroxylamin-Derivaten der Formel III, vorzugsweise mit Säureadditionssalzen davon, umsetzt. Diese Reaktion erfolgt zweckmässigerweise in einem unter den Reaktionsbedingungen inerten basischen Lösungsmittel, beispielsweise in tertiären Aminen, wie z.B. Pyridin, Triäthylamin und dergleichen. Man kann die Reaktion aber auch in einem Alkohol, wie z.B. Methanol, Äthanol und dergleichen, oder in Mischungen davon mit Wasser durchführen, und zwar in Gegenwart eines säurebindenden Mittels, wie z.B. Kalium- und Natriumcarbonat und dergleichen. Die Reaktionstemperatur ist nicht kritisch, man kann demnach von etwa Raumtemperatur bis Siedetemperatur des Reaktionsgemisches arbeiten, wobei letzteres bevorzugt ist.

Gemäss Verfahrensvariante b) können Benzophenonoxim-Derivate der allgemeinen Formel I hergestellt werden, indem man Verbindungen der Formel Ia hydrolysiert. Die für diese Reaktion notwendigen Reaktionsbedingungen können von jedem Fachmann leicht ermittelt werden. Zweckmässigerweise hydrolysiert man mit wässriger Natron- oder Kalilauge, in einem Temperaturbereich von etwa Raumtemperatur bis etwa 100°, vorzugsweie bei etwa 50°.

Gemäss Verfahrensvariante c) können Benzophenonoxim-Derivate der Formel I hergestellt werden, indem man Verbindungen der Formel Ib am (an den) Oxim-Sauerstoffatom(en) alkyliert. Diese Umsetzung kann mit jedem geeigneten Alkylierungsmittel durchgeführt werden, beispielsweise mit einem entsprechenden Halogenid, wie z.B. Methyljodid und Äthyljodid, oder einem Dialkylsulfat, wie z.B. Dimethylsulfat. Die Reaktion erfolgt zweckmässigerweise in einem inerten organischen Lösungsmittel, z. B. in einem Äther, wie Tetrahydrofuran, Dioxan oder Dimethoxyäthan, oder in anderen aprotisch dipolaren Lösungsmitteln, in Gegenwart eines säurebindenden Mittels, wie z.B. Natrium- oder Kaliumhydroxid oder dergleichen. Vorzugsweise verwendet man eine kleine Menge eines quaternären Ammoniumsalzes, wie z.B. Tetra-n-butyl-ammoniumbromid, als Katalysator. Die Reaktionstemperatur liegt zweckmässigerweise bei etwa Raumtemperatur, kann aber auch darüber liegen.

Gemäss Verfahrensvariante d) können Benzophenonoxim-Derivate der allgemeinen Formel I hergestellt werden, indem man Verbindungen der Formel IV mit Hydroxylamin-Derivaten der Formel III umsetzt. Die für diese Reaktion notwendigen Bedingungen können von jedem Fachmann leicht ermittelt werden, beispielsweise kann man in Analogie zu Verfahrensvariante a) arbeiten, d.h. in Analogie zur Umsetzung von Verbindungen der allgemeinen Formel II mit Verbindungen der Formel III.

Gemäss Verfahrensvariante e) können Benzophenonoxim-Derivate der allgemeinen Formel I in pharmazeutisch annehmbare Säureadditionssalze übergeführt werden. Die Herstellung solcher Salze erfolgt nach allgemein üblichen Methoden. Es kommen sowohl Salze mit anorganischen als auch Salze mit organischen Säuren in Betracht, beispielsweise Hydrochloride, Hydrobromide, Sulfate, Citrate, Acetate, Succinate, Methansulfonate, p-Toluolsulfonate und dergleichen.

Die als Ausgangsstoffe verwendeten Verbindungen der Formeln II und IV gehören an sich bekannten Stoffklassen an, nicht spezifisch vorbeschriebene Vertreter können nach bekannten und jedem Fachmann geläufigen Methoden hergestellt werden. Beispielsweise können Verbindungen der allgemeinen Formel II, worin $R^7$ einen Rest der Formel $R^3–O–N=C(R^6)–$ bedeutet, aus den entsprechenden Verbindungen der allgemeinen Formel II, worin $R^7$ einen Rest der Formel $R^6–CO–$ bedeutet, hergestellt werden, und zwar durch Behandeln mit einer Verbindung der Formel III unter milden Bedingungen, beispielsweise durch Umsetzen einer Verbindung der Formel II, worin $R^7$ einen Rest der Formel $R^6–CO–$ bedeutet, mit Hydroxylamin-hydrochlorid in Pyridin oder

einem Gemisch davon mit Äthanol bei Raumtemperatur.

Überraschenderweise hat es sich gezeigt, dass die neuen Benzophenonoxim-Derivate der eingangs definierten allgemeinen Formel I ausgeprägte aldosteronantagonistische Eigenschaften aufweisen. Diese aldosteronantagonistischen Eigenschaften lassen sich, wie nachstehend erläutert, an adrenalektomierten Ratten nachweisen.

Verabreicht man adrenalektomierten Ratten Aldosteron, so beobachtet man – im Vergleich zu unbehandelten Tieren – eine ausgeprägte Verminderung der Natrium-Ausscheidung (Natrium-Retention), eine erhöhte Kalium-Ausscheidung (Kalium-Exkretion) sowie eine Verminderung des ausgeschiedenen Harnvolumens. Gibt man den Tieren vor der Behandlung mit Aldosteron Verbindungen der Formel I, so beobachtet man – im Vergleich zu nur mit Aldosteron behandelten Tieren (Kontrolltiere) – eine ausgeprägte Erhöhung der Natrium-Ausscheidung (das heisst: die durch Aldosteron bewirkte Natrium-Retention wird antagonisiert), wogegen die Kalium-Exkretion und das Harnvolumen in geringerem Ausmass beeinflusst werden.

Der Standardversuch wird wie folgt durchgeführt:

Weibliche Holtzmann-Ratten (150–180 g) werden 70 bis 74 Stunden vor Versuchsbeginn bilateral adrenalektomiert. Nach der Operation erhalten die Tiere ein gebräuchliches Ratten-Trokkenfutter und 0,9%-ige Kochsalzlösung zum Trinken. 16–17 Stunden vor Versuchsbeginn wird den Tieren das Futter entzogen, wogegen sie nach wie vor adlibitum 0,9%-ige Kochsalzlösung trinken können. Bei Versuchsbeginn wird den Tieren die auf Aldosteronantagonismus zu prüfende Substanz mittels einer Magensonde verabreicht. 30 Minuten später erhalten die Tiere eine subcutane Injektion von 4 mmg/kg Aldosteron. Nach weiteren 90 Minuten werden die Harnblasen der Tiere durch sorgsältiges suprapubisches Drücken entleert, worauf die Tiere ohne Nahrung und ohne Nahrung und ohne Getränk einzeln in Metabolismuskäfige verbracht werden. Der Urin der Tiere wird dann während 3 Stunden gesammelt, worauf ihre Harnblasen nochmals entleert werden. Der spontan ausgeschiedene Harn und der am Schluss des Versuches durch Ausdrücken der Harnblasen erhaltene Restharn werden in graduierten Zentrifugengläsern gesammelt. Natrium- und Kalium-Konzentrationen des Harns werden mit einem Flammenphotometer bestimmt.

In der nachfolgenden Tabelle werden die Resultate dargestellt, welche mit repräsentativen Vertretern der durch die allgemeine Formel I definierten Verbindungsklasse in dem vorstehend geschilderten Versuch erhalten worden sind. Angegeben werden in dieser Tabelle für jede der darin figurierenden Verbindungen die verabreichte Dosis (in mg/kg p.o.) sowie die prozentuale Veränderung des Harnvolumens, der Natrium-Ausscheidung und der Kalium-Ausscheidung im Vergleich zu den Kontrolltieren (das heisst im Vergleich zu den nur mit Aldosteron behandelten Tieren). Ausserdem enthält die Tabelle Angaben über die akute Toxizität der untersuchten Verbindungen (DL 50 in mg/kg bei einmaliger oraler Verabreichung an Mäuse).

Toxizität und Wirkung an der adrenalektomierten Ratte

| $R^1$ | $R^2$ | $R^3$ | $R^4$ | $R^5$ | Dosis mg/kg p.o. | Volumen | $[Na^+]$ | $[K^+]$ | DL 50 mg/kg p.o. |
|---|---|---|---|---|---|---|---|---|---|
| | | | | | | in %, bezogen auf Kontrolltiere, | | | |
| H | $-COCH_2NH_2$ | OH | Cl | $-NO_2$ | 0,1 | 114 | 211 | 99 | >5000 |
| H | $-COCH_2NH_2$ | OH | H | $-NO_2$ | 0,1 | 152 | 230 | 97 | >5000 |
| $CH_3$ | $-COCH_2NH_2$ | OH | Cl | $NH_2$ | 1 | 155 | 254 | 94 | >5000 |
| H | $-COCH_2NH_2$ | OH | F | $CH_3-C=N-OH$ | 1 | 156 | 327 | 102 | >5000 |
| | | | | | 0,01 | 161 | 215 | 86 | |

Die Benzophenonoxim-Derivate der allgemeinen Formel I und ihre pharmazeutisch annehmbaren Säureadditionssalze können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von Tabletten, Lacktabletten, Dragées und Hartgelatinekapseln können die Benzophenonoxim-Derivate der allgemeinen Formel I und ihre pharmazeutisch annehmbaren Säureadditionssalze mit pharmazeutisch inerten, anorganischen oder organischen Excipientien verabreicht werden. Als solche Excipientien kann man für Tabletten, Dragées und Hartgelatinekapseln z.B. Lactose, Maisstärke oder Derivate davon, Talk, Stearinsäure oder deren Salze etc. verwenden.

Für Weichgelatinekapseln eignen sich als Excipientien z.B. pflanzliche Oele, Wachse, Fette, halbfeste und flüssige Polyole etc.; je nach Beschaffenheit des Wirkstoffes sind jedoch bei Weichgelatinekapseln überhaupt keine Excipientien erforderlich.

Zur Herstellung von Lösungen und Sirupen eignen sich als Excipientien z.B. Wasser, Polyole,

Saccharose, Invertzucker, Glukose und dergleichen.

Für Injektionslösungen eignen sich als Excipientien z.B. Wasser, Alkohole, Polyole, Glyzerin, pflanzliche Öle etc..

Für Suppositorien eignen sich als Excipientien z.B. natürliche oder gehärtete Öle, Wachse, Fette, halbflüssige oder flüssige Polyole und dergleichen.

Die pharmazeutischen Präparate können daneben noch Konservierungsmittel, Lösevermittler, Stabilisierungsmittel, Netzmittel, Emulgiermittel, Süssmittel, Färbemittel, Aromatisierungsmittel, Salze zur Veränderun des osmotischen Druckes, Puffer, Überzugsmittel oder Antioxidantien enthalten. Sie können auch noch andere therapeutisch wertvolle Stoffe enthalten.

Wie eingangs erwähnt sind Arzneimittel, enthaltend eine oder mehrere Verbindungen der allgemeinen Formel I oder pharmazeutisch annehmbare Säureadditionssalze davon, ebenfalls Gegenstand der vorliegenden Erfindung, weiterhin auch ein Verfahren zur Herstellung solcher Arzneimittel, welches dadurch gekennzeichnet ist, dass man eine oder mehrere Verbindungen der allgemeinen Formel I oder pharmazeutisch annehmbare Säureadditionssalze davon und gegebenenfalls einen oder mehrere andere therapeutische wertvolle Stoffe in eine galenische Darreichungsform bringt. Ein weiterer Gegenstand der vorliegenden Erfindung ist – wie eingangs erwähnt – die Verwendung von Benzophenonoxim-Derivaten der allgemeinen Formel I und von pharmazeutisch annehmbaren Säureadditionssalzen davon bei der Bekämpfung bzw. Verhütung von Krankheiten, insbesondere bei der Bekämpfung bzw. Verhütung von Herzversagen, von hepatitischer Aszites, von primärem Aldosteronismus und von essentieller Hypertonie. Die Dosierung kann innerhalb weiter Grenzen variieren und ist natürlich in jedem einzelnen Fall den individuellen Gegebenheiten anzupassen. In allgemeinen dürfte bei oraler Verabreichung eine Tagesdosis von etwa 20 mg bis 2500 mg angemessen sein.

In den nachfolgenden Beispielen, welche die vorliegende Erfindung näher erläutern, ihren Umfang jedoch in keiner Weise beschränken sollen, sind sämtliche Temperaturen in Celsiusgraden angegeben.

Beispiel 1

Eine Lösung von 50 g (0,167 Mol) 5-(2-Fluorphenyl)-1,3-dihydro-7-nitro-2H-1,4-benzodiazepin-2-on und 17,4 g (0,25 Mol) Hydroxylamin-hydrochlorid in 200 ml Pyridin wird über Nacht unter Argon zum Rückfluss erhitzt. Das Reaktionsgemisch wird im Rotationsverdampfer eingeengt und der Rückstand in Methylenchlorid aufgenommen. Die organische Phase wird mit Wasser gewaschen, getrocknet und eingedampft. Die wässrige Phase wird über Nacht in einem Perforator mit Chloroform extrahiert. Nach Trocknen und Entfernen des Lösungsmittels wird der Rückstand mit dem oben erhaltenen Rohprodukt vereinigt, in 200 ml Methylenchlorid suspendiert und abfiltriert. Man erhält 2-Amino-2'-[o-fluor-α-(hydroxyimino)benzyl]-4'-nitro-acetanilid vom Schmelzpunkt 198°.

Beispiel 2

Eine Lösung von 10 g (35,55 mMol) 1,3-Dihydro-7-nitro-5-phenyl-2H-1,4-benzodiazepin-2-on und 3,7 g (53,3 mMol) Hydroxylamin-hydrochlorid in 80 ml Pyridin wird während 1 Stunde unter Rückfluss erhitzt. Nach dem Erkalten wird das Reaktionsgemisch in Methylenchlorid aufgenommen, mit Wasser gewaschen, getrocknet und eingedampft. Das verbleibende Pyridin wird im Vakuum azeotrop mit Toluol entfernt. Das feste Rohprodukt wird in heissem tert.-Butylmethyläther suspendiert und abfiltriert. Man erhält 2-Amino-2'-[α-(hydroxyimino)-benzyl]-4'-nitro-acetanilid vom Schmelzpunkt 167° (Zers.).

Beispiel 3

Aus 10 g (31,7 mMol) 5-(o-Chlorphenyl)-1,3-dihydro-7-nitro-2H-1,4-benzodiazepin-2-on erhält man in Analogie zur Arbeitsvorschrift von Beispiel 2 2-Amino-2'-[o-chlor-α-(hydroxyimino) -benzyl] -4'-nitro-acetanilid vom Schmelzpunkt 177° (Zers.).

Beispiel 4

In eine Lösung von 5,25 g (15,05 mMol) 2-Amino-2'-[o-chlor-α-(hydroxyimino)benzyl]-4'-nitro-acetanilid in 300 ml Methylenchlorid und 100 ml Äthanol leitet man während 10 Minuten Chlorwasserstoff ein. Die Lösung wird im Vakuum zur Trockene eingedampft und der Rückstand über nacht im Vakuum bei 60° getrocknet. Man erhält 2-Amino-2'-[o-chlor-α-(hydroxyimino)benzyl]-4'-nitro-acetanilidhydrochlorid vom Schmelzpunkt 140° (Zers.).

Beispiel 5

In eine Lösung von 2,7 g (8,59 mMol) 2-Amino-2'-[α-(hydroxyimino)benzyl]-4'-nitro-acetanilid in 150 ml Methylenchlorid und 30 ml Äthanol leitet man während 10 Minuten Chlorwasserstoff ein. Die Lösung wird anschliessend zur Trockene eingedampft und der Rückstand über Nacht im Vakuum bei 60° getrocknet. Man erhält 2-Amino-2'-[α-(hydroxyimino)benzyl]-4'-nitro-acetanilid-hydrochlorid vom Schmelzpunkt 150° (Zers.).

Beispiel 6

Eine Lösung von 50 g (0,168 Mol) 7-Acetyl-5-(o-fluorphenyl)-1,3-dihydro-2H-1,4-benzodiazepin-2-on und 50 g (0,719 Mol) Hydroxylamin-hydrochlorid in 400 ml Pyridin rührt man 21 Stunden bei Raumtemperatur, engt im Vakuum ein und entfernt das restliche Pyridin azeotrop durch mehrmaliges Abdampfen mit Toluol. Zum Rückstand gibt man 300 ml Wasser und stellt das pH der Lösung auf ca. 7 ein. Der dabei entstandene Niederschlag wird abfiltriert, mit Wasser gewaschen und im Vakuum über Phosphorpentoxid

bei 60° getrocknet. Man erhält 2-Amino-2'-[o-fluor-α-(hydroxiimino)benzyl]-4'-[1-hydroxiimino) äthyl] acetanilid- hydrochlorid vom Schmelzpunkt 219–220°.

Beispiel 7

10 g (26,26 mMol) 2-Amino-2'-[o-fluor-α-(hydroxyimino)benzyl-4'-[1-(hydroxyimino) äthyl-acetanilid werden während 1 Stunde bei 50° mit 3N Natronlauge behandelt. Man stellt das pH der Lösung auf 8 ein, filtriert das ausgefallene Material ab, wäscht es mit Wasser und trocknet im Vakuum über Phosphorpentoxid bei 50°. Man erhält 2-Amino-2'-fluor-5- [1-(hydroxyimino) äthyl]benzophenon-oxim als Schaum vom Schmelzpunkt ca. 165°.

Beispiel 8

a) Zu einer Lösung von 50 g (0,158 Mol) 5-(o-Chlor-phenyl)- 1,3-dihydro- 7-nitro- 2H-1,4-benzodiazepin-2-on in 600 ml Aceton gibt man 42,4 g Kaliumcarbonat und 18,5 ml Methyljodid und rührt das Gemisch während 16 Stunden bei Raumtemperatur. Nach Abfiltrieren von unlöslichem Material wird das Filtrat eingedampft und der Rückstand in Methylenchlorid aufgenommen. Die organische Phase wird einmal mit Wasser gewaschen, getrocknet und eingedampft, wobei 5- (o-Chlorphenyl)- 1, 3-dihydro- 1-methyl-7-nitro-2H-1,4-benzodiazepin-2-on vom Schmelzpunkt 220° resultiert.

b) Zu einer eisgekühlten Lösung von 50 g (0,152 Mol) 5-(o-Chlorphenyl)-1,3-dihydro-1-methyl- 7-nitro- 2H- 1,4-benzodiazepin- 2-on in 500 ml konz. Salzsäure gibt man unter Rühren portionenweise insgesamt 103 g Zinnchloriddihydrat so zu, dass die Reaktionstemperatur unterhalb 85° bleibt. Man rührt noch 3 Stunden bei Raumtemperatur, neutralisiert in der Kälte mit 10N Natronlauge und extrahiert die Suspension in einem Perforator. Zuerst während 24 Stunden mit 1,5 l Äthanol/Chloroform (1:9) und dann über Nacht mit 1,5 l Äthanol/1,2-Dichloräthan (1:4). Nach dem Eindampfen der Extrakte wird der Rückstand aus Äthanol umkristallisiert, wobei man 7-Amino-5(o-chlorphenyl)-1,3-dihydro-1-methyl- 2H-1,4- benzodiazepin- 2-on vom Schmelzpunkt 238–239° erhält.

Beispiel 9

Eine Lösung von 5,0 g (16,68 mMol) 7-Amino-5-(o-chlorphenyl)-1,3-dihydro-1-methyl-2H-1,4-benzodiazepin-2-on und 1,74 g (22,02 mMol) Hydroxylamin-hydrochlorid in 50 ml Pyridin wird 2 Stunden unter Argon zum Rückfluss erhitzt. Nach dem Erkalten wird das Reaktionsgemisch in 600 ml Chloroform/Äthanol (4:1) aufgenommen und mit 250 ml Wasser gewaschen. Die wässrige Phase wird auf pH 8 eingestellt, mit Kochsalz gesättigt und über Nacht im Perforator mit Chloroform extrahiert. Nach dem Trocknen und Eindampfen des Lösungsmittels wird der Rückstand in heissem tert.-Butyl-methyläther suspendiert und abfiltriert. Man erhält 2,4'-Diamino-2'-[o-chlor-α-(hydroxyimino)benzyl]-N-methylacetanilid vom Schmelzpunkt 150° (Zers.).

Beispiel 10

Eine Lösung von 3,0 g (14,15 mMol) 2-Amino-benzophenon-oxim in 120 ml Tetrahydrofuran versetzt man nacheinander mit 1,07 g Kaliumhydroxid, 100 mg Tetrabutylammoniumbromid, 1,81 ml Dimethylsulfat und rührt das Gemisch über Nacht bei Raumtemperatur. Das Reaktionsgemisch wird in Methylenchlorid aufgenommen, mit Wasser gewaschen, getrocknet und eingedampft. Der Rückstand wird zweimal in siedendem Äther suspendiert und abfiltriert und ergibt 2- Amino-benzophenon- O-methyl- oxim vom Schmelzpunkt 138°.

Beispiel A

2-Amino- 2'-[o-chlor-α- (hydroxyimino) benzyl]-4'-nitro-acetanilid kann wie folgt als Wirkstoff zur Herstellung von pharmazeutischen Präparaten verwendet werden:

| a) Tabletten | 1 Tablette enthält |
|---|---|
| Wirkstoff | 200 mg |
| mikrokristalline Cellulose | 155 mg |
| Maisstärke | 25 mg |
| Talk | 25 mg |
| Hydroxypropylmethylcellulose | 20 mg |
| | 425 mg |

Der Wirkstoff wird mit der Hälfte der mikrokristallinen Cellulose gemischt und mit einer 10-proz. Lösung von Hydroxypropylmethylcellulose in einem Gemisch von Isopropanol und Methylenchlorid granuliert. Das Granulat wird getrocknet, gesiebt und mit dem Rest der Hilfsstoffe gemischt. Alsdann wird es auf einer Presse zu biplanen Tabletten von 12 mm Durchmesser mit Kreuzbruchrille verpresst.

| b) Kapseln | 1 Kapsel enthält |
|---|---|
| Wirkstoff | 100,0 mg |
| Maisstärke | 20,0 mg |
| Milchzucker | 95,0 mg |
| Talk | 4,5 mg |
| Magnesiumstearat | 0,5 mg |
| | 220,0 mg |

Der Wirkstoff wird mit den Hilfsstoffen gemischt und gesiebt. Nach erneutem Mischen wird die erhaltene Kapselfüllmasse auf einer vollautomatischen Kapselabfüllmaschine in Gelatinesteckkapseln geeigneter Grösse abgefüllt.

**Patentansprüche für die Vertragsstaaten: BE-CH-DE-FR-GB-IT-LU-NL-SE-LI**

1. Benzophenon-Derivate der allgemeinen Formel

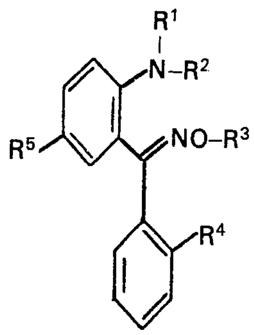

I

worin $R^1$ Wasserstoff oder niederes Alkyl, $R^2$ Wasserstoff oder Aminoacetyl, $R^3$ Wasserstoff oder niederes Alkyl, $R^4$ Wasserstoff oder Halogen, $R^5$ Wasserstoff, Amino, Nitro oder einen Rest der Formel $R^3$–ON=C($R^6$)- und $R^6$ niederes Alkyl bedeuten, mit der Massgabe, dass $R^5$ einen Rest der Formel H–ON=C($R^6$)- bedeutet, wenn $R^2$ und $R^3$ beide Wasserstoff sind, und pharmazeutisch annehmbare Säureadditionssalze von Verbindungen der allgemeinen Formel I.

2. Verbindungen gemäss Anspruch 1, dadurch gekennzeichnet, dass $R^1$ Wasserstoff oder Methyl, $R^2$ Aminoacetyl, $R^3$ Wasserstoff oder Methyl und $R^4$ Wasserstoff, Fluor oder Chlor bedeuten.

3. 2-Amino-2'- [o-fluor-α- (hydroxyimino) benzyl]-4'-[1-(hydroxyimino)äthyl]acetanilid.

4. 2-Amino-2'- [o-chlor-α- (hydroxyimino) benzyl] -4'-nitro-acetanilid, 2-Amino-2'- [α-(hydroxyimino) benzyl]-4'- nitro-acetanilid, 2,4'-Diamino-2'- [o-chlor-α- (hydroxyimino)-benzyl] -N-methyl-acetanilid, 2-Amino-2'- [o-fluor-α- (hydroxyimino) benzyl]-4'-nitro- acetanilid; 2-Amino-2'-fluor-5- [1- (hydroxyimino) äthyl] benzophenon-oxim und 2-Amino-benzophenon-O-methyl-oxim.

5. Verbindungen gemäss einem der Ansprüche 1 bis 4 als pharmazeutische Wirkstoffe.

6. Verbindungen gemäss einem der Ansprüche 1 bis 4 als aldosteronantagonistische Wirkstoffe.

7. Verfahren zur Herstellung von Verbindungen, gemäss einem der Ansprüche 1 bis 4, dadurch gekennzeichnet, dass man

a) ein Benzodiazepin-Derivat der allgemeinen Formel

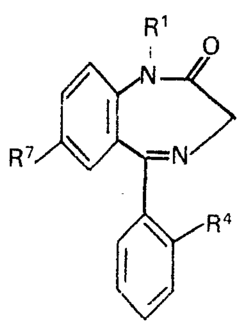

II

worin $R^7$ Wasserstoff, Amino, Nitro oder einen Rest der Formel $R^3$–ON=C($R^6$)– oder O=C($R^6$)– bedeutet, und $R^1$, $R^3$, $R^4$ und $R^6$ die in Anspruch 1 angegebene Bedeutung besitzen, mit einer Verbindung der allgemeinen Formel

$R^3$–$ONH_2$ III

worin $R^3$ die in Anspruch 1 angegebene Bedeutung besitzt, umsetzt, oder

b) eine Verbindung der allgemeinen Formel

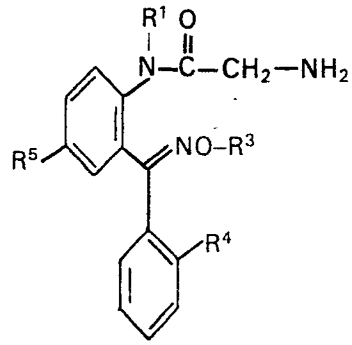

Ia

worin $R^1$, $R^3$, $R^4$ und $R^5$ die in Anspruch 1 ange-Bedeutung besitzen, mit der Massgabe, dass $R^5$ einen Rest der Formel H–ON=C($R^6$)– bedeutet, wobei $R^6$ die in Anspruch 1 angegebene Bedeutung besitzt, wenn $R^3$ Wasserstoff ist, hydrolysiert, oder

c) eine Verbindung der allgemeinen Formel

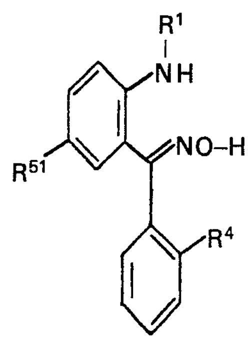

Ib

worin $R^{51}$ Wasserstoff, Amino, Nitro oder einen Rest der Formel H–ON=C ($R^6$)– bedeutet, und $R^1$, $R^4$ und $R^6$ die in Anspruch 1 angegebene Bedeutung besitzen, am (an den) Oxim-Sauerstoffatom(en) alkyliert, oder

d) ein Benzophenon-Derivat der allgemeinen Formel

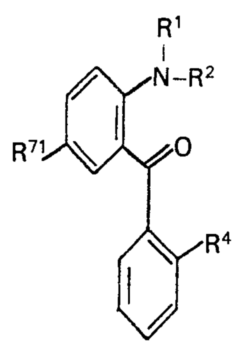

IV

worin $R^{71}$ Wasserstoff, Amino, Nitro oder einen Rest der Formel $O=\underset{R^6}{\underset{|}{C}}-$ bedeutet, und $R^1$, $R^2$, $R^4$ und $R^6$ die in Anspruch 1 angegebene Bedeutung besitzen, mit einer Verbindung der allgemeinen Formel

$R^3-ONH_2$ III

worin $R^3$ die in Anspurch 1 angegebene Bedeutung besitzt, umsetzt, und erwünschtenfalls

e) ein Benzophenonoxim-Derivat der allgemeinen Formel I in ein pharmazeutisch annehmbares Säureadditionssalz überführt.

8. Arzneimittel, enthaltend eine Verbindung gemäss einem der Ansprüche 1 bis 4.

9. Aldosteronantagonisierende Mittel, enthaltend eine Verbindung gemäss einem der Ansprüche 1 bis 4.

**Patentansprüche für den Vertragsstaat: AT**

1. Verfahren zur Herstellung von Benzophenon-Derivaten der allgemeinen Formel

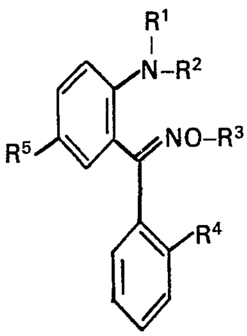

I

worin $R^1$ Wasserstoff oder niederes Alkyl, $R^2$ Wasserstoff oder Aminoacetyl, $R^3$ Wasserstoff oder niederes Alkyl, $R^4$ Wasserstoff oder Halogen, $R^5$ Wasserstoff, Amino, Nitro oder einen Rest der Formel $R^3-ON=C(R^6)-$ und $R^6$ niederes Alkyl bedeuten, mit der Massgabe, dass $R^5$ einen Rest der Formel $H-ON=C(R^6)-$ bedeutet, wenn $R^2$ und $R^3$ beide Wasserstoff sind, und von pharmazeutisch annehmbaren Säureadditionssalzen davon, dadurch gekennzeichnet, dass man

a) ein Benzodiazepin-Derivat der allgemeinen Formel

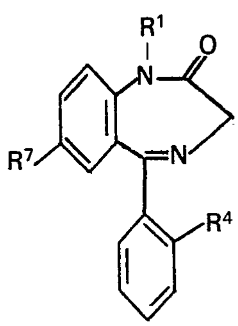

II

worin $R^7$ Wasserstoff, Amino, Nitro oder einen Rest der Formel $R^3-ON=C(R^6)-$ oder $O=C(R^6)-$ bedeutet, und $R^1$, $R^3$, $R^4$ und $R^6$ obige Bedeutung besitzen, mit einer Verbindung der allgemeinen Formel

$R^3-ONH_2$ III

worin $R^3$ obige Bedeutung besitzt, umsetzt, oder

b) eine Verbindung der allgemeinen Formel

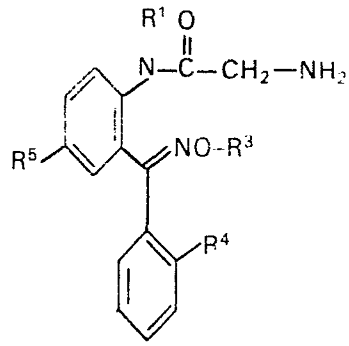

Ia

worin $R^1$, $R^3$, $R^4$ und $R^5$ obige Bedeutung besitzen, mit der Massgabe, dass $R^5$ einen Rest der Formel $H-ON=C(R^6)-$ bedeutet, worin $R^6$ obige Bedeutung besitzt, wenn $R^3$ Wasserstoff ist, hydrolysiert, oder

c) eine Verbindung der allgemeinen Formel

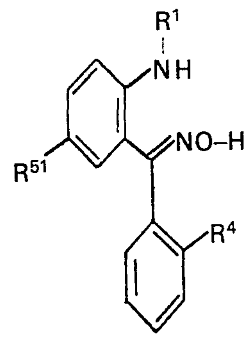

Ib

worin $R^{51}$ Wasserstoff, Amino, Nitro oder einen Rest der Formel $H-ON=C(R^6)-$ bedeutet, und $R^1$, $R^4$ und $R^6$ obige Bedeutung besitzen, am (an den) Oxim-Sauerstoffatom(en) alkyliert, oder

d) ein Benzophenon-Derivat der allgemeinen Formel

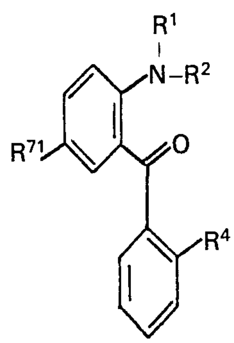

IV

worin $R^{71}$ Wasserstoff, Amino, Nitro oder einen Rest der Formel $O=\underset{R^6}{C}-$ bedeutet, und $R^1$, $R^2$, $R^4$ und $R^6$ obige Bedeutung besitzen, mit einer Verbindung der allgemeinen Formel

$R^3-ONH_2$ III

worin $R^3$ obige Bedeutung besitzt, umsetzt, und erwünschtenfalls

e) ein Benzophenonoxim-Derivat der allgemeinen Formel I in ein pharmazeutisch annehmbares Säureadditionssalz überführt.

2. Verfahren gemäss Anspruch 1, dadurch gekennzeichnet, dass man eine Verbindung der in Anspruch 1 definierten allgemeinen Formel I herstellt, worin $R^1$ Wasserstoff oder Methyl, $R^2$ Aminoacetyl, $R^3$ Wasserstoff oder Methyl und $R^4$ Wasserstoff, Fluor oder Chlor bedeuten.

3. Verfahren gemäss Anspruch 1 oder 2, dadurch gekennzeichnet, dass man 2-Amino-2'-[o-fluor-α-(hydroxyimino)-benzyl]4'-[1-(hydroxyimino)äthyl]acetanilid herstellt.

**Revendications pour les Etats contractants: BE, CH, DE, FR, GB, IT, LU, NL, SE, LI.**

1. Dérivés de benzophénone de formule générale

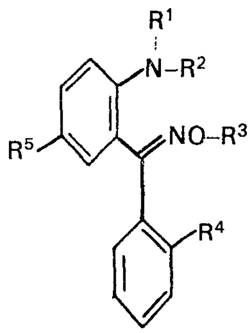

I

où $R^1$ représente un hydrogène ou un alcoyle inférieur, $R^2$ un hydrogène ou un aminoacétyle, $R^3$ un hydrogène ou un alcoyle inférieur, $R^4$ un hydrogène ou un halogène, $R^5$ un hydrogène, un amino, un nitro ou un radical de formule $R^3$–ON=C($R^6$)– et $R^6$ un alcoyle inférieur, avec la précision que $R^5$ représente un radical de formule H–ON=C ($R^6$)–, lorsque $R^2$ et $R^3$ sont tous deux des hydrogènes, et les sels d'addition d'acides pharmaceutiquement acceptables des composés de formule générale I.

2. Composés selon la revendication 1, caractérisés en ce que $R^1$ représente un hydrogène ou un méthyle, $R^2$ un aminoacétyle, $R^3$ un hydrogène ou un méthyle et $R^4$ un hydrogène, un fluor ou un chlore.

3. 2-Amino-2'- [o-fluoro-α- (hydroxyimino) benzyl] -4'-[1- (hydroxyimino) éthyl]acétanilide.

4. 2-Amino-2'- [o-chloro-α-(hydroxyimino) benzyl] -4'-nitro-acétanilide, 2-amino-2'- [α-(hydroxyimino) benzyl]-4'-nitro-acétanilide, 2,4'-diamino-2'- [o-chloro-α- (hydroxyimino) benzyl] -N-méthyl-acétanilide, 2-amino-2'- [o-fluoro-α-(hydroxyimino) benzyl]-4'-nitro-acétanilide; 2-amino-2'-fluoro-5- [1-hydroxyimino) éthyl] benzophénoneoxime et 2-amino- benzophénone-O- méthyl- oxime.

5. Composés selon l'une des revendications 1 à 4 comme substances actives pharmaceutiques.

6. Composés selon l'une des revendications 1 à 4 comme substances actives antagonistes de l'aldostérone.

7. Procédé de préparation de composés selon l'une des revendications 1 à 4, caractérisé en ce que a) on fait réagir un dérivé de benzodiazépine de formule générale

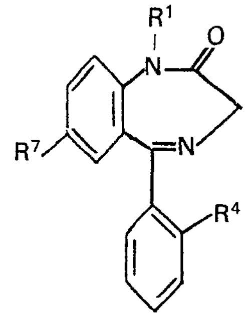

II

où $R^7$ représente un hydrogène, un amino, un nitro ou un radical de formule $R^3$–ON=C($R^6$)– ou O=C($R^6$)–, et $R^1$, $R^3$, $R^4$ et $R^6$ ont la signification donnée dans la revendication 1, avec un composé de formule générale

$R^3$–$ONH_2$ III

où $R^3$ a la signification donnée dans la revendication 1, ou

b) on hydrolyse un composé de formule générale

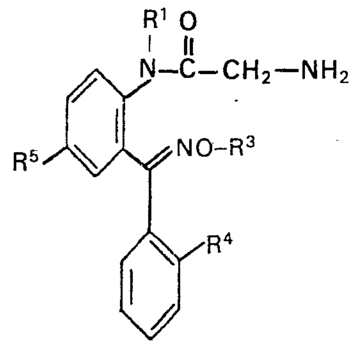

Ia

où $R^1$, $R^3$, $R^4$ et $R^5$ ont la signification donnée dans la revendication 1, avec la précision que $R^5$ représente un radical de formule H–ON=C($R^6$)–, où $R^6$ a la signification donnée dans la revendication 1 lorsque $R^3$ est un hydrogène, ou

c) on alcoyle un composé de formule générale

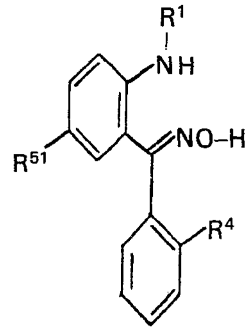

Ib

où $R^{51}$ représente un hydrogène, un amino, un nitro ou un radical de formule H–ON=C($R^6$)– et $R^1$, $R^4$ et $R^6$ ont la signification donnée dans la revendication 1, sur le ou les atome(s) d'oxygène de l'oxime, ou

d) on fait réagir un dérivé de benzophénone de formule générale

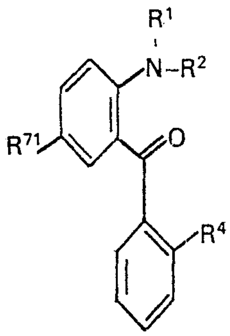

IV

où $R^{71}$ représente un hydrogène, un amino, un nitro ou un radical de formule $O=\underset{R^6}{\underset{|}{C}}-$, et $R^1$, $R^2$, $R^4$ et $R^6$ ont la signification donnée dans la revendication 1, avec un composé de formule générale

$R^3$–$ONH_2$ III

où $R^3$ a la signification donnée dans la revendication 1, et si on le désire

e) on transforme un dérivé de benzophénonoxime de formule générale I en un sel d'addition d'acide pharmaceutiquement acceptable.

8. Médicaments contenant un composé selon l'une des revendications 1 à 4.

9. Agents antagonistes de l'aldostérone contenant un composé selon l'une des revendications 1 à 4.

**Revendications pour l'Etat contractant: AT**

1. Procédé de préparation de dérivés de benzophénone de formule générale

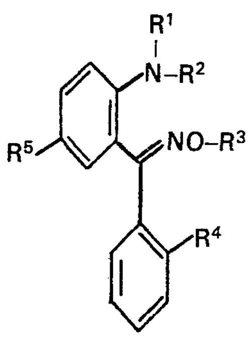

I

où $R^1$ représente un hydrogène ou un alcoyle inférieur, $R^2$ un hydrogène ou un aminoacétyle, $R^3$ un hydrogène ou un alcoyle inférieur, $R^4$ un hydrogène ou un halogène, $R^5$ un hydrogène, un amino, un nitro ou un radical de formule $R^3$–$ON=C(R^6)$– et $R^6$ un alcoyle inférieur, avec la précision que $R^5$ représente un radical de formule H–$ON=C(R^6)$– lorsque $R^2$ et $R^3$ sont tous deux des hydrogènes, et de leurs sels d'addition d'acides pharmaceutiquement acceptables, caractérisés en ce que

a) on fait réagir un dérivé de benzodiazépine de formule générale

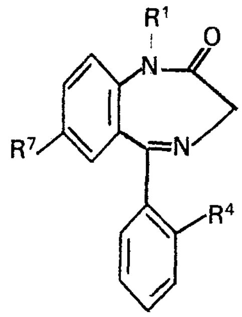

II

où $R^7$ représente un hydrogène, un amino, un nitro ou un radical de formule $R^3$–$ON=C(R^6)$ – ou $O=C(R^6)$–, et $R^1$, $R^3$, $R^4$ et $R^6$ ont la signification donnée ci-dessus, avec un composé de formule générale

$R^3$–$ONH_2$ III

où $R^3$ a la signification donnée ci-dessus, ou

b) on hydrolyse un composé de formule générale

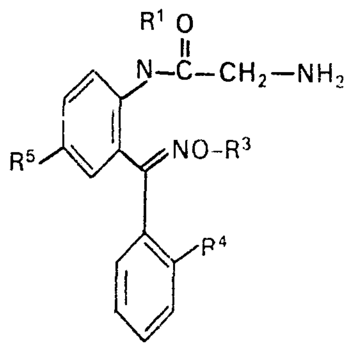

Ia

où $R^1$, $R^3$, $R^4$ et $R^5$ ont la signification donnée ci-dessus, avec la précision que $R^5$ représente un radical de formule H–$ON=C(R^6)$– où $R^6$ a la signification donnée ci-dessus lorsque $R^3$ est un hydrogène, ou

c) on alcoyle un composé de formule générale

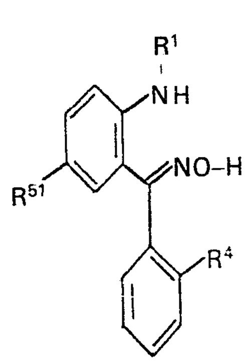

Ib

où $R^{51}$ représente un hydrogène, un amino, un nitro ou un radical de formule H–$ON=C(R^6)$–, et $R^1$, $R^4$ et $R^6$ ont la signification donnée ci-dessus, sur le ou les atome(s) d'oxygène de l'oxime, ou

d) on fait réagir un dérivé de benzophénone de formule générale

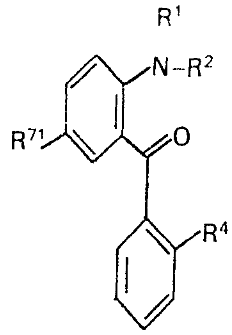

IV

où $R^{71}$ représente un hydrogène, un amino, un nitro ou un radical de formule $O = \underset{\substack{| \\ R^6}}{C}-$ et $R^1$, $R^2$, $R^4$ et $R^6$ ont la signification donnée ci-dessus, avec un composé de formule générale

$R^3$–$ONH_2$ III

où $R^3$ a la signification donnée ci-dessus, et si on le désire

e) on transforme un dérivé de benzophénon-oxime de formule générale I en un sel d'addition d'acide pharmaceutiquement acceptable.

2. procédé selon la revendication 1, caractérisé en ce qu'on prépare un composé de formule générale I définie dans la revendication 1, où $R^1$ représente un hydrogène ou un méthyle, $R^2$ un aminoacétyle, $R^3$ un hydrogène ou un méthyle et $R^4$ un hydrogène, un fluor ou un chlore.

3. Procédé selon la revendication 1 ou 2, caractérisé en ce qu'on prépare le 2-amino-2'-[o-fluoro-α-(hydroxyimino)-benzyl]-4'-[1-(hydroxyimino)éthyl]acétanilide.

**Claims for the Contracting States: BE, CH, DE, FR, GB, IT, LU, NL, SE, LI.**

1. Benzophenone derivatives of the general formula

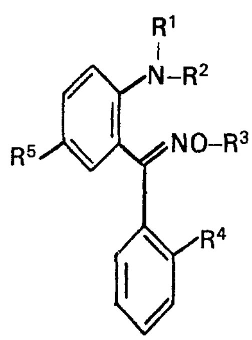

I

wherein $R^1$ signifies hydrogen or lower alkyl, $R^2$ signifies hydrogen or aminoacetyl, $R^3$ signifies hydrogen or lower alkyl, $R^4$ signifies hydrogen or halogen, $R^5$ signifies hydrogen, amino, nitro or a residue of the formula $R^3$–$ON = C(R^6)$– and $R^6$ signifies lower alkyl, with the proviso that $R^5$ signifies a residue of the formula H–$ON = C(R^6)$– when $R^2$ and $R^3$ are both hydrogen, and pharmaceutically acceptable acid addition salts of compounds of general formula I.

2. Compounds according to claim 1, characterized in that $R^1$ signifies hydrogen or methyl, $R^2$ signifies aminoacetyl, $R^3$ signifies hydrogen or methyl and $R^4$ signifies hydrogen, fluorine or chlorine.

3. 2-Amino-2'-[o-fluoro-α-(hydroxyimino) benzyl]-4'-[1-(hydroxyimino)ethyl]acetanilide.

4. 2-Amino-2'-[o-chloro-α-(hydroxyimino) benzyl]-4'-nitro-acetanilide, 2-amino-2'-[α-(hydroxyimino)benzyl]-4'-nitro-acetanilide, 2,4'-diamino-2'-[o-chloro-α-(hydroxyimino) benzyl]-N-methyl-acetanilide, 2-amino-2'-[o-fluoro-α-(hydroxyimino)benzyl]- 4'-nitro- acetanilide; 2-amino- 2'-fluoro- 5-[1- (hydroxyimino) ethyl] benzophenone oxime and 2-amino-benzophenone O-methyl oxime.

5. Compounds according to any one of claims 1 to 4 as pharmaceutically active substances.

6. Compounds according to any one of claims 1 to 4 as aldosterone-antagonistically active substances.

7. Process for the manufacture of compounds according to any one of claims 1 to 4, characterized by

a) reacting a benzodiazepine derivative of the general formula

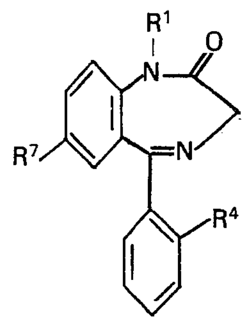

II

wherein $R^7$ signifies hydrogen, amino, nitro or a residue of the formula $R^3$–$ON = C(R^6)$– or $O = C(R^6)$– and $R^1$, $R^3$, $R^4$ and $R^6$ have the significance given in claim 1, with a compound of the general formula

$R^3$–$ONH_2$ III

wherein $R^3$ has the significance given in claim 1, or

b) hydrolyzing a compound of the general formula

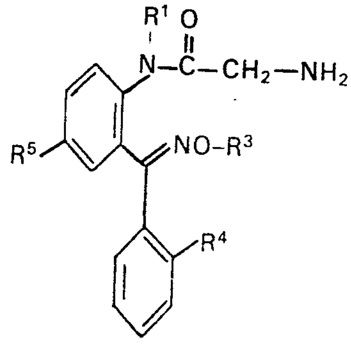

Ia

wherein $R^1$, $R^3$, $R^4$ and $R^5$ have the significance given in claim 1, with the proviso that $R^5$ signifies a residue of the formula $H\text{–}ON=C(R^6)\text{–}$, in which $R^6$ has the significances given in claim 1, when $R^3$ is hydrogen, or

c) alkylating a compound of the general formula

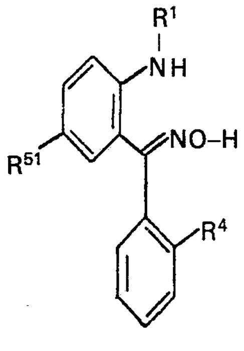

Ib

wherein $R^{51}$ signifies hydrogen, amino, nitro or a residue of the formula $H\text{–}ON=C(R^6)\text{–}$ and $R^1$, $R^4$ and $R^6$ have the significance given in claim 1, at the oxime oxygen atom(s), or

d) reacting a benzophenone derivative of the general formula

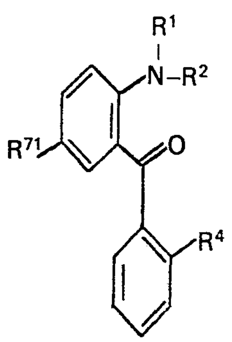

IV

wherein $R^{71}$ signifies hydrogen, amino, nitro or a residue of the formula $O=\underset{R^3}{\underset{|}{C}}-$ and $R^1$, $R^2$, $R^4$ and $R^6$ have the significance given in claim 1, with a compound of the general formula

$R^3\text{–}ONH_2$ III

wherein $R^3$ has the significance given in claim 1, and, if desired,

e) converting a benzophenone oxime derivative of general formula I into a pharmaceutically acceptable acid addition salt.

8. Medicaments, containing a compound according to any one of claims 1 to 4.

9. Aldosterone-antagonizing medicaments, containing a compound according to any one of claims 1 to 4.

**Claims for the Contracting State: AT**

1. Process for the manufacture of benzophenone derivatives of the general formula

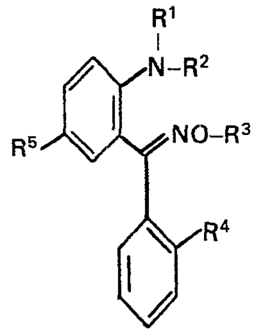

I

wherein $R^1$ signifies hydrogen or lower alkyl, $R^2$ signifies hydrogen or aminoacetyl, $R^3$ signifies hydrogen or lower alkyl, $R^4$ signifies hydrogen or halogen, $R^5$ signifies hydrogen, amino, nitro or a residue of the formula $R^3\text{–}ON=C(R^6)\text{–}$ and $R^6$ signifies lower alkyl, with the proviso that $R^5$ signifies a residue of the formula $H\text{–}ON=C(R^6)\text{–}$ when $R^2$ and $R^3$ are both hydrogen, and of pharmaceutically acceptable acid addition salts thereof, characterized by

a) reacting a benzodiazepine derivative of the general formula

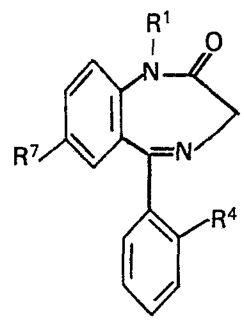

II

wherein $R^7$ signifies hydrogen, amino, nitro or a residue of the formula $R^3\text{–}ON=C(R^6)\text{–}$ or $O=C(R^6)\text{–}$ and $R^1$, $R^3$, $R^4$ and $R^6$ have the above significance, with a compound of the general formula

$R^3\text{–}ONH_2$ III

wherein $R^3$ has the above significance, or

b) hydrolyzing a compound of the general formula

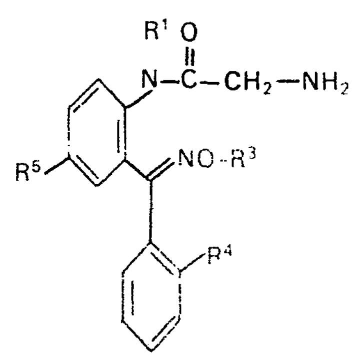

Ia

wherein $R^1$, $R^3$, $R^4$ and $R^5$ have the above significance, with the proviso that $R^5$ signifies a residue of the formula $H\text{–}ON=C(R^6)\text{–}$, in which $R^6$ has the above signigicance, when $R^3$ is hydrogen, or

c) alkylating a compound of the general formula

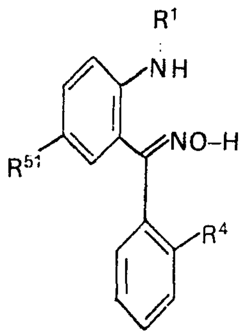

Ib

wherein $R^{51}$ signifies hydrogen, amino, nitro or a residue of the formula $H-ON=C(R^6)-$ and $R^1$, $R^4$ and $R^6$ have the above significance, at the oxime oxygen atom(s), or

d) reacting a benzophenone derivative of the general formula

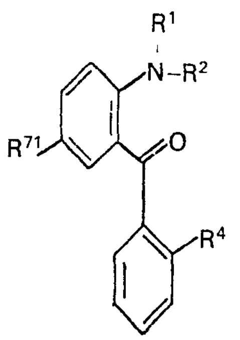

IV

wherein $R^{71}$ signifies hydrogen, amino, nitro or a residue of the formula $O=\underset{R^6}{\underset{|}{C}}-$ and $R^1$, $R^2$, $R^4$ and $R^6$ have the above significance, with a compound of the general formula

$R^3-ONH_2$ III

wherein $R^3$ has the above significance, and, if desired,

e) converting a benzophenone oxime derivative of general formula I into a pharmaceutically acceptable acid addition salt.

2. Process according to claim 1, characterized in that a compound of general formula I defined in claim 1 wherein $R^1$ signifies hydrogen or methyl, $R^2$ signifies aminoacetyl, $R^3$ signifies hydrogen or methyl and $R^4$ signifies hydrogen, fluorine or chlorine is manufactured.

3. Process according to claim 1 or 2, characterized in that 2-amino-2′- [o-fluoro-α- (hydroxyimino)benzyl] -4′-[1-(hydroxyimino)ethyl] acetanilide is manufactured.